(19) Europäisches Patentamt
European Patent Office
Office européen des brevets

(11) EP 1 424 085 A1

(12) **EUROPEAN PATENT APPLICATION**

(43) Date of publication:
**02.06.2004 Bulletin 2004/23**

(51) Int Cl.[7]: **A61L 15/28**

(21) Application number: **03254080.9**

(22) Date of filing: **27.06.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **26.11.2002 US 305040**

(71) Applicant: **ETHICON, INC.**
**Somerville, NJ 08876 (US)**

(72) Inventor: **Pendharkar, Sanyog Manohar**
**Old Bridge, NJ 08857 (US)**

(74) Representative: **Mercer, Christopher Paul et al**
**Carpmaels & Ransford**
**43, Bloomsbury Square**
**London WC1A 2RA (GB)**

# (54) Hemostatic wound dressing containing aldehyde-modified polysaccharide

(57) The present invention is directed to hemostatic wound dressings that include a substrate for contacting and/or covering a wound, where the substrate includes a wound-contacting surface, and which substrate contains or is fabricated at least in part from a biocompatible, aldehyde-modified polysaccharide, preferably biodegradable polysaccharides and to methods of providing hemostasis to a wound, which method includes applying to a wound requiring hemostasis the hemostatic wound dressing of the present invention.

EP 1 424 085 A1

## Description

### FIELD OF THE INVENTION

The present invention relates to wound dressings, e.g. hemostatic wound dressings, containing or fabricated in part from an aldehyde-modified, polysaccharide, e.g. a biodegradable aldehyde-modified regenerated cellulose, and to methods of using such wound dressings.

### BACKGROUND OF THE INVENTION

The control of bleeding is essential and critical in surgical procedures to minimize blood loss, to reduce post-surgical complications, and to shorten the duration of the surgery in the operating room. Oxidized regenerated cellulose, as described herein below and commonly referred to as ORC, due to its biodegradability, bactericidal, and hemostatic properties, has long been used as a hemostatic wound dressing in a variety of surgical procedures, including neurosurgery, abdominal surgery, cardiovascular surgery, thoracic surgery, head and neck surgery, pelvic surgery, and skin and subcutaneous tissue procedures. ORC, as recognized heretofore by those skilled in the art of wound dressings, is carboxylic-oxidized, regenerated cellulose comprising reactive carboxylic acid groups. In other words, the oxidized cellulose is carboxyl-modified to contain a certain amount of carboxylic acid moieties. Examples of hemostatic ORC absorbable hemostats commercially available include Surgicel® absorbable hemostat, a knitted fabric of ORC; Surgicel Nu-Knit® absorbable hemostat, a dense ORC fabric; and Surgicel® Fibrillar absorbable hemostat; all available from Johnson & Johnson Wound Management Worldwide, a division of Ethicon, Inc., Somerville, New Jersey, a Johnson & Johnson Company. Other examples of commercial absorbable hemostats containing carboxyl-oxidized cellulose include Oxycel® absorbable cellulose surgical wound dressing, available from Becton Dickinson, Morris Plains, New Jersey.

Conventional oxidized cellulose (OC) and oxidized regenerated cellulose (ORC) hemostats noted above are knitted or non-woven fabrics comprising carboxylic acid groups, as noted above, in amounts effective to provide them with anti-microbial properties. However, the acid-based ORC and OC, due to their acidic pH, also rapidly denatures acid-sensitive, hemostatic proteins, including thrombin or fibrinogen, on contact. Thus it is most problematic and has not been suggested in the art to use the OC or ORC as a carrier for acid-sensitive species, such as thrombin and fibrinogen, as well as other acid-sensitive biologics and pharmaceutical agents.

Cellulose can undergo many modifications in oxidizing media and, accordingly, the properties of oxidized cellulose, generally referred to as oxycellulose, vary widely depending on the oxidation agents used. Oxidation of the secondary alcohol groups of cellulose with periodic acid or its salts to form a aldehyde-modified cellulose has been disclosed in the prior art as a means of characterizing the chemical structure of mono-, oligo- and polysaccharide-based materials.

To date, aldehyde-modified cellulose has not been utilized in wound dressings to provide hemostasis. Methods of producing highly oxidized tri-carboxylic acid derivatives of cellulose as hemostic materials, involving two-stage oxidation by successive processing with an iodine-containing compound and nitrogen oxides, has been disclosed in RU2146264 and IN159322. As disclosed in these disclosures, oxidized cellulosic materials were prepared by preliminary oxidation with metaperiodate or periodic acid to yield periodate-oxidized, dialdehyde cellulose to form the intermediate for forming OC. The dialdehyde cellulose intermediate then is further oxidized by $NO_2$ to yield the OC with a higher carboxylic acid content, which is suitable for use as a hemostatic, anti-microbial and wound healing agent. The disclosures do not, however, suggest or disclose that the periodate-oxidized, dialdehyde cellulose intermediate formed in the first stage oxidation may or should be used in the preparation of wound dressings, e.g. hemostatic wound dressings.

The use of cotton gauze that has been modified by oxidation to contain aldehyde, and then further modified by carboxymethylation, sulfonation or phosphorylation, has been disclosed for use in wound dressings. However, such dressings are not known to be hemostatic and contain functional groups such as carboxymethly, sulfonyl or phosphonyl that are acidic.

It would be advantageous to provide a hemostatic wound dressing that not only provides hemostasis and anti-microbial properties similar to conventional OC-containing hemostatic wound dressings, but that also is compatible with acid-sensitive species, such as thrombin and fibrinogen. To date, no such wound dressing has been reported. The present invention provides such a wound dressing that not only provides hemostatic and anti-microbial properties equivalent to conventional ORC-based hemostatic wound dressings, but that also is compatible with "acid-sensitive" species.

It further would be an advantage to provide wound dressings that are anti-microbial, that aid in covering and protecting a wound, and that preferably are essentially free of unnecessary or undesirable reactive moieties.

## SUMMARY OF THE INVENTION

The present invention is directed to hemostatic wound dressings that include a substrate for contacting a wound, which substrate comprises a wound-contacting surface, and which substrate comprises and is fabricated at least in part from a biocompatible, aldehyde-modified polysaccharide. The aldehyde-modified polysaccharide preferably comprises an amount of aldehyde moieties effective to render the substrate biodegradable. The wound dressings provide and maintain effective hemostasis to a wound in need of hemostasis. The invention also is directed to methods of providing hemostasis to a wound, which method includes applying to a wound requiring hemostasis the wound dressing described herein.

## DETAILED DESCRIPTION OF THE INVENTION

The present invention is directed to hemostatic wound dressings that provide and maintain effective hemostasis when applied to a wound requiring hemostasis. Effective hemostasis, as used herein, is the ability to control and/or abate capillary, venous, or arteriole bleeding within an effective time, as recognized by those skilled in the art of hemostasis. Preferably, when tested for effective hemostasis as described herein the wound dressings of the present invention provide hemostasis within about fifteen minutes, and more preferably twelve minutes, from the time the wound dressing is applied to the wound.

The hemostatic dressings of the present invention are particularly useful when conventional procedures to control and/or abate bleeding, such as pressure or suturing, are either ineffective or impractical. The hemostats of the present invention also are useful

where one desires to utilize in, or in conjunction with, the wound dressing hemostatic agents, or other biological or therapeutic compounds, moieties or species, particularly those "acid-sensitive" adjuncts that may be degraded or denatured by, or otherwise detrimentally affected by acidic pH provided by, e.g. carboxylic acid moieties, such as is provided by conventional hemostats.

The wound dressings may take various physical forms and may include, without limitation, fibrous or non-fibrous, woven or non-woven dressings. In preferred embodiments, the wound dressing may comprise a fiber, including microfibers, a film, a fabric, a foam, a bead, a gel, or combinations thereof. Regardless of the form of the wound dressing, it will comprise a substrate for contacting and/or covering the wound.

In certain embodiments, substrates, may be incorporated into slurries, pastes, dispersions or other mixtures and applied directly to a wound surface. In such instances, additional wound dressings may be applied over such substrates, but are not necessarily required. In some embodiments, the wound dressing could comprise the substrate in a carrier for delivery of the substrate to the wound. For example, polymeric beads, microfibers, or ground foam substrates, all comprising the aldehyde-modified polysaccharides of the present invention, may be dispersed in slurries or dispersions that may be applied directly to a wound or, in certain circumstances, injected subcutaneously or otherwise disposed internally into the body to provide hemostasis. Gels also may be formulated so as to be applied to a wound or otherwise disposed within the body in order to provide hemostasis.

In certain wound dressings, the dressing may consist essentially of the substrate, or may consist of the substrate. This is particularly true where the wound dressing is fabricated from a knitted, woven or non-woven hemostatic fabric that has been oxidized to provide aldehyde modification, as described herein, and which serves as the substrate for the wound dressing. In those cases, while the wound dressing may further include such components as backing layers, adhesive layers, or the like, the wound dressing can include only the hemostatic fabric.

The wound dressing substrate will comprise a wound-contacting surface. Such substrates may take various physical forms, including, but not limited to, fibrous or non-fibrous, woven or non-woven substrates. In certain embodiments, the wound dressing substrates may comprise a fiber, including microfibers, a film, a fabric, a foam, a bead, a gel, or combinations thereof. In preferred embodiments, the substrate comprises a knitted fabric. The fabric may be formed, cut or otherwise shaped to cover the wound surface, thereby providing protection of the wound from physical trauma and effective hemostasis of the wound.

Wound dressings of the present invention, and more particularly the wound-contacting substrates thereof, comprise a biocompatible, aldehyde-modified polysaccharide. In preferred wound dressings, the polysaccharide will contain an amount of aldehyde moieties effective to render the modified polysaccharide biodegradable, meaning that the polysaccharide is degradable by the body into components that either are resorbable by the body, or that can be passed readily by the body. More particularly, the biodegraded components do not elicit permanent chronic foreign body reaction because they are absorbed by the body, such that no permanent trace or residual of the component is retained at the implantation site.

Aldehyde-modified polysaccharides used in the present invention may be prepared from biocompatible polysaccharides that are useful in medical devices. Such polysaccharides include, without limitation, cellulose, alkyl cellulose, e.g. methylcellulose, alkylhydroxyalkyl cellulose, hydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparan, heparan sulfate, dermatan sulfate, keratan sulfate, carrageenans, chitosan, starch, amylose, amylopectin, poly-N-glucosamine, polymannuronic acid, polyglucuronic acid polyguluronic acid, and derivatives of any of the above. In preferred embodiments, the polysaccharide is oxidized as described herein to assure that the aldehyde-modified polysaccharide is biodegradable. Such biodegradable, aldehyde-modified polysaccharides may be represented by Structure I below.

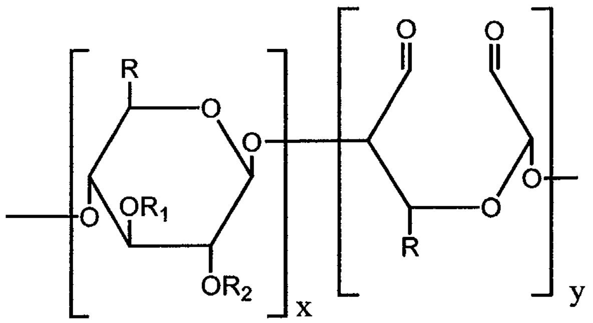

x+y = 100%

where x and y represent mole percent, x plus y equals 100 percent, x is from about 95 to about 5,
y is from about 5 to about 95; and
R may be $CH_2OR_3$, $COOR_4$, sulphonic acid, or phosphonic acid; $R_3$ and $R_4$ may be H, alkyl, aryl, alkoxy or aryloxy, and $R_1$ and $R_2$ may be H, alkyl, aryl, alkoxy, aryloxy, sulphonyl or phosphoryl.

In preferred embodiments of the present invention, the biocompatible, biodegradable hemostatic wound dressing comprises a wound contacting/covering substrate prepared from a biocompatible, biodegradable, aldehyde-modified regenerated cellulose. Regenerated cellulose is preferred due to its higher degree of uniformity versus cellulose that has not been regenerated. Regenerated cellulose is described in, for instance, United States Patent 3,364,200, the contents of which is hereby incorporated by reference as if set forth in its entirety.

In particular, preferred aldehyde-modified regenerated cellulose is one comprising repeating units of Structure II:

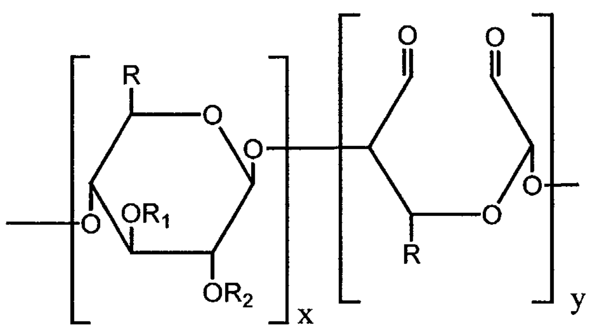

x+y = 100%

where x and y represent mole percent, x plus y equals 100 percent, x is from about 95 to about 5,
y is from about 5 to about 95; and R is $CH_2OH$, R, and $R_2$ are H.

In certain embodiments according to the present invention, x is from about 90 to 10 and y is about 10 to about

90. Preferably, x is from about 80 to 20 and y is from about 20 to about 80. Even more preferably, x is from about 70 to about 30. Most preferably, x is about 70 and y is about 30.

The hemostatic dressings of the present invention also provide anti-microbial activities due to the presence of effective amounts of the aldehyde moieties. It has been shown that in spite of being non-acidic, the aldehyde-modified regenerated cellulose is anti-microbial in nature. The hemostats of the present invention were found to be significantly effective against microorganisms, such as Methicillin-resistant *Staphylococcus aureus* (MRSA) *and Pseudomonas aeruginosa*, etc. The anti-microbial activities of the non-acidic aldehyde-modified regenerated cellulose are shown to be comparable to those of the acidic carboxylic oxidized regenerated cellulose conventionally used. The acidic carboxylic oxidized regenerated cellulose loses its anti-microbial activities upon neutralization reaction or over a period of time as the acid groups are neutralized in the body. However, the aldehyde-modified regenerated cellulose utilized in the present invention is expected to retain its anti-microbial activity over a longer period of time.

In preferred embodiments of the invention, the aldehyde-modified regenerated polysaccharide is essentially free of functional or reactive moieties other than aldehyde moieties. By essentially free, it is meant that the polysaccharide does not contain such functional or reactive moieties in amounts effective to alter the properties of the aldehyde-modified polysaccharide or to provide the substrate comprising the polysaccharide with a pH of less than about 4.5, more preferably less than about 5, or greater than about 9, preferably about 9.5. Such moieties include, without limitation, carboxylic acid moieties typically present on wound dressings made from OC. Excess levels of carboxylic acid moieties will lower the pH of the substrates and dressings so that they are not compatible for use with those species that may be degraded or denatured by such a low pH, e.g. thrombin. Other undesired moieties include, without limitation, sulfonyl or phosphonyl moieties.

The hemostat of the present invention exhibits increased thermal stability compared to that of the carboxylic oxidized regenerated cellulose fabric (ORC). The increased thermal stability may be indicative of improved physical shelf-life, compared to ORC or neutralized ORC.

In certain embodiments of the invention, the fabrics utilized in the present invention may be knitted, woven, or non-woven, provided that the fabric possesses the physical properties adequate for wound dressings, in general, and hemostasic wound dressings, specifically. Fabrics oxidized by periodic acid or its salts described in the present invention are expected to retain physical properties and mechanical integrity required for use in wound dressings. Hemostatic fabrics useful for use in hemostatic wound dressings according to the present invention include fabrics comprising the aldehyde-modified polysaccharides of the present invention. Structures that are useful in the present invention include those described in United States Patent Numbers 2,773,000, 3,364,200 and 4,626,253, the contents each of which is hereby incorporated by reference herein as if set forth in its entirety. Also useful in wound dressings of the present invention are fabrics useful in adhesion prevention such as those described in United States Patent No. 5,002,551, the contents of which is hereby incorporated by reference herein as if set forth in its entirety.

In certain embodiments of the invention, the hemostatic wound dressing of the present invention comprises as the wound contacting/covering hemostatic substrate a warp knitted tricot fabric constructed of bright rayon yarn that has been oxidized by periodic acid or its salts such that the substrate comprises aldehyde moieties. Both Scanning Electron Microscopic (SEM) images and fabric mechanical properties indicate that the physical characteristics (density, thickness) and physical performance, e.g. fabric tensile strength and mullen burst strength, of the aldehyde-modified regenerated cellulose in the present invention are comparable to those of the fabric disclosed in United States Patent 4,626,253.

The hemostat of the present invention remains very flexible, conforms to a bleeding site, and retains good tensile and compressive strength to withstand handling during application. The aldehyde-modified regenerated cellulose hemostat can be cut into different sizes and shapes to fit the surgical needs. It can be rolled up or packed into irregular anatomic areas.

Other warp knit tricot fabric constructions which produce equivalent physical properties may, of course, be utilized in the manufacture of the aldehyde-modified regenerated cellulose hemostatic wound dressings of the present invention, and such constructions will be apparent to those skilled in the art once having the benefit of this disclosure.

In certain embodiments of the invention, a biologics, a drug, or a combination of pharmaceutical agents, including those that otherwise may be sensitive to the low pH of conventional OC-containing wound dressings, may be incorporated into certain wound dressings of the present invention without having to adjust pH prior to incorporation into the dressing. To fabricate such a hemostatic wound dressing, a drug or agent first may be dissolved in an appropriate solvent. The fabric is then coated with the drug solution and the solvent is removed. Preferred biologics, drugs and agent include analgesics, anti-infective agents, antibiotics, adhesion preventive agents, pro-coagulants, and wound healing growth factors. The aldehyde groups formed on the polysaccharide matrix during the periodate oxidation reaction can be used to covalently bond amine containing biologics and therapeutic agents. The combination of such biologics, drugs and agents with wound dressings of the present invention using the aldehyde-modified regenerated cellulose substrates can provide improved hemostatic wound dressings, wound healing dressings, drug delivery devices, and tissue engineering matrices.

While the following examples demonstrate certain embodiments of the invention, they are not to be interpreted as limiting the scope of the invention, but rather as contributing to a complete description of the invention.

Example 1:

Preparation of woven aldehyde-modified regenerated cellulose fabric:

A 15.75 g piece of Nu-Knit® rayon fabric was cut in the form of a strip 1.5 inches wide. The strip was wound on a mandrel and suspended in 600 ml of aqueous isopropyl alcohol (IPA) (200 ml IPA/400 ml de-ionized (DI) water). 20.8 g of sodium periodate (Aldrich, Milwaukee, 53201) was dissolved in the solution (1:1 molar ratio) and the mandrel was rotated at moderate rpm in the solution for 21 hours at ambient temperature. It is essential that the oxidation of the fabric be conducted in the dark. The solution pH was 3.8. The solution was discarded after the reaction. The mandrel with the oxidized fabric was washed for 30 minutes in 1 liter of cold DI water containing 50 ml of ethylene glycol. It was then washed with aqueous IPA (50/50) for 15 minutes, followed by a pure IPA wash for 15 minutes. The fabric was dried in ambient air for several hours. [Aldehyde content: Ave. 22.83%]

The oxidized fabric then was evaluated for hemostasis as set forth below. Results are provided in Table 1.

Example 2:

Preparation of non-woven aldehyde-modified cellulose fabric:

A 10 g piece of cellulose rayon non-woven fabric was cut in the form of a rectangle and placed in an aqueous solution of sodium periodate (Aldrich, Milwaukee, 53201) (1:0.7 molar ratio). The fabric was placed in a container modified to exclude light and soaked in the dark for 24 hours at 37°C. The solution was discarded after the reaction. The fabric was repeatedly washed with DI water until the pH was 6-7. It was then washed with aqueous IPA (50/50) for 15 minutes. The fabric then was washed in pure IPA for 15 minutes. The fabric was dried in ambient air for several hours. [aldehyde content: 51.04%]

The oxidized fabric then was evaluated for hemostasis as set forth below. Results are provided in Table 1.

Example 3:

Preparation of aldehyde-modified regenerated cellulose Powders:

10.6 g of powdered cellulose rayon was suspended in an aqueous solution of sodium periodate (Aldrich, Milwaukee, 53201)(13.9 g in 250 ml DI water) and stirred for 7 hours at ambient temperature in the dark. The solution was filtered after the reaction. The filtrate was repeatedly washed with DI water until the pH was in the range of from 6 to 7. It was then washed with aqueous IPA (50/50) and pure IPA for 15 min each. The powder was dried in air for several hours. [aldehyde content: 32.8 %]

The oxidized powder then was evaluated for hemostasis as set forth below. Results are provided in Table 1.

Example 4:

Preparation of aldehyde-modified cellulose beads:

13.67 g of porous cellulose beads are floated in an aqueous solution of sodium periodate (Aldrich, Milwaukee, 53201) (18g in 250 ml DI water/125ml IPA) and stirred for 24 hours at ambient temperature. The material was filtered and the filtrate (beads and crushed beads) was repeatedly washed with DI water until the pH was in the range of from 6 to 7. It was then washed with aqueous IPA (50/50) and pure IPA for 15 min each. The material was dried in air for several hours. [aldehyde content: intact beads—29.86 %; crushed beads—35%] The oxidized beads then were evaluated for hemostasis as set forth below. Results are provided in Table 1.

Example 5:

Preparation of water-soluble aldehyde-modified methylcellulose:

100 g of a 5% methylcellulose (Aldrich, Milwaukee, 53201) aqueous solution was combined with 3 g of periodic acid (Aldrich, Milwaukee, 53201) and was then stirred for 5 hours at ambient temperature in the dark. 1.5 ml of ethylene glycol was added to the reaction solution and stirred for 30 minutes. 2000 ml of acetone were added slowly into the

reaction solution to precipitate the methylcellulose dialdehyde. The reaction mixture was allowed to stand for 20-30 minutes to separate the liquid phase from the solid phase. The supernatant then was removed and the solid phase centrifuged to precipitate the solids. The solid precipitate was dissolved in 100 ml DI over night followed by dialysis for 72 hours. The final wet mixture was lyophilized to form a sponge/foam.

The material then was evaluated for hemostasis as set forth below. Results are provided in Table 1.

Example 6:

Preparation of water-soluble aldehyde-modified sodium carboxymethyl cellulose:

100 g of a 4% sodium carboxymethyl cellulose (Aldrich, Milwaukee, 53201) aqueous solution was added with 2.12 g of sodium periodate (Aldrich, Milwaukee, 53201) and was then stirred for 24 hours at 4°C in the refrigerator. 200 ml ethanol was slowly added into the reaction solution to precipitate the aldehyde-modified carboxymethyl cellulose. The supernatant was removed and the solid phase mixture centrifuged to precipitate the solids. The solid precipitate was dissolved in 100 ml DI. Precipitation and centrifuging were repeated. The final product was dissolved in 100 ml DI then lyophilized to form a sponge/foam.

The material then was evaluated for hemostasis as set forth below. Results are provided in Table 1.

Table 1

| Sample | Time to Hemostasis (seconds) |
|---|---|
| Example 1 fabric | 187 (n=11) |
| Example 2 | 96 (n=5) |
| Example 3 | 120 (n=3) |
| Example 4 | 238 (n=1) |
| Example 5 | 120 (n=2) |
| Example 6 | 360 (n=2) |
| Surgical gauze | >720 (n=6) |

Example 7:

Hemostatic evaluation of dressings in porcine splenic incision model:

A porcine spleen incision model was used for hemostasis evaluation. The materials were cut into 2.5 cm X 2.0 cm rectangles. A linear incision of 1.5 cm with a depth of 1.0 cm was made with a surgical blade on a porcine spleen. After application of the test article, digital tamponade was applied to the incision for 2 minutes. The hemostasis was then evaluated. Additional applications of digital tamponade for 30 seconds each were used until complete hemostasis was achieved. The fabrics providing hemostasis within 15 minutes, preferably within 12 minutes, were considered to be effective hemostats. Wound dressings comprising aldehyde-modified regenerated cellulose achieved rapid hemostasis compared to the negative control of surgical gauze.

Example 8:

Characterization of Aldehyde Content of aldehyde modified regenerated cellulose

1 g of fabric was digested in 10 ml of 0.5N NaOH and 100 ml DI water at ambient temperature for 2 hours. The residual NaOH was back-titrated to a fixed endpoint using 0.1N HCl. The aldehyde content was calculated using the formula:

$$\% \text{ Aldehyde Content} = [(B-S)*0.81]/W$$

where B is the burette reading(in ml) from a blank titration, S is the burette reading (in ml) from a sample and W is the sample weight. Aldehyde content is the number of glucose rings (by mole) containing the di-aldehyde functionality.

Aldehyde content of aldehyde modified regenerated cellulose in the present invention, processed in various

physical forms, is substantially consistent in the range of 20-50%.

Example 9:

Effects of aldehyde-modified regenerated cellulose and ORC absorbable hemostat on Methicillin-resistant *Staphylococcus aureus* (MRSA)

Studies were designed to study the effect of the sample against the microbial challenge over a period of time. Aldehyde-modified regenerated cellulose fabric (Example 1), Surgicel NU-KNIT® and Surgicel Fibrillar® samples were cut to a weight of 165-mg (+/-2-mg). The weighed sample was then placed into a sterile test tube. An aliquot of 11-ml of TSB was added to the test tube containing the sample. Three (3) test tubes were prepared for each aldehyde-modified regenerated cellulose powder and control sample. Each test tube was innoculated with 0.1-µL of microorganism from stock cultures. Prepared samples were subsequently diluted and pour plated. This procedure was carried out at time 0 (immediately following innoculation), 1, 6, 24, and 48 hours after innoculation.

Methicillin-resistant *Staphylococcus aureus* (MRSA) organisms were grown in Trypticase Soy Broth (TSB) for 24 hours at 30-35°C. Trypticase Soy Agar (TSA) and TSB were the media used in this study. The dilutions used 0.85% saline. All media and solutions were sterile.

The experiment results demonstrate that periodate oxidized regenerated cellulose hemostats (Example 1 fabric) and the Surgicel Nu-Knit® controls were both effective at significantly reducing the microbial populations of MRSA.

**Claims**

1. A hemostatic wound dressing, comprising:

   a substrate for contacting a wound, said substrate comprising;
   a wound-contacting surface; and
   a biocompatible, aldehyde-modified polysaccharide, wherein said wound dressing is hemostatic.

2. The wound dressing of claim 1 wherein said substrate comprises a fiber, a fabric, a sponge, a foam, a film, a bead, a gel, or combinations thereof.

3. The wound dressing of claim 1 or 2 wherein said aldehyde-modified polysaccharide is selected from the group consisting of aldehyde-modified cellulose, alkylcellulose, alkylhydroxyalkyl cellulose, hydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparan, heparan sulfate, dermatan sulfate, keratan sulfate, carrageenans, chitosan, starch, amylose, amylopectin, poly-N-glucosamine, polymannuronic acid, polyglucuronic acid, polyguluronic acid and derivatives of any of the above.

4. The wound dressing of any preceding claim wherein said aldehyde-modified polysaccharide comprises an amount of aldehyde moieties effective to render the substrate biodegradable.

5. The wound dressing of any preceding claim wherein said aldehyde-modified polysaccharide comprises regenerated cellulose.

6. The wound dressing of any preceding claim wherein said substrate comprises a fabric.

7. The wound dressing of claim 6 wherein said regenerated cellulose comprises repeating units of structure II:

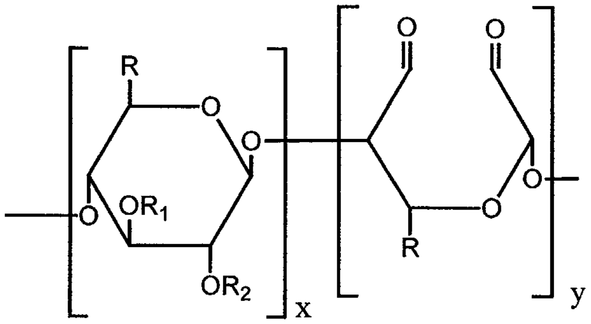

x+y = 100%

where x and y represent mole percent, x plus y equals 100 percent, x is from 95 to 5, y is from 5 to 95; and R is $CH_2OH$, R, and $R_2$ are H.

**8.** The wound dressing of claim 7 wherein x is from 80 to 20 and y is from 20 to 80.

**9.** The wound dressing of claim 8 wherein x is from 70 to 30 and y is from 30 to 70.

**10.** The wound dressing of claim 8 wherein x is about 70 and y is about 30.

**11.** The wound dressing of any preceding claim wherein said aldehyde-modified polysaccharide is essentially free of carboxylic acid moieties.

**12.** The wound dressing of claim 1 wherein said substrate consists essentially of said aldehyde-modified polysaccharide.

**13.** The wound dressing of claim 1 wherein said wound dressing provides hemostasis of a wound after application of said wound dressing to said wound.

**14.** The wound dressing of claim 1 consisting essentially of said substrate.

**15.** Use, in the manufacture of a wound dressing for providing hemostasis to a wound, of a hemostatic wound dressing according to any one of claims 1 to 14.

European Patent Office

# EUROPEAN SEARCH REPORT

Application Number
EP 03 25 4080

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 3 868 955 A (SIRAGUSA JUDITH ANN ET AL) 4 March 1975 (1975-03-04)<br>* column 1, line 4 - line 8 *<br>* column 2, line 14 - line 31 *<br>* column 3, line 23 - line 27 *<br>* column 3, line 35 - column 4, line 15 *<br>* column 5, line 22 - line 65 *<br>--- | 1-4,6, 11-15 | A61L15/28 |
| D,X | US 3 364 200 A (MOSER CHARLES E ET AL) 16 January 1968 (1968-01-16)<br>* column 1, line 56 - column 2, line 34 *<br>* column 3, line 5 - line 51 *<br>* column 3, line 68 - column 4, line 39 *<br>* column 4, line 71 - column 5, line 25 *<br>* column 12, line 45 - line 67 *<br>* claims 1-4 *<br>--- | 1-6, 12-15 | |
| D,X | US 4 626 253 A (BROADNAX JR CECIL H) 2 December 1986 (1986-12-02)<br>* column 1, line 5 - line 31 *<br>* column 1, line 63 - column 2, line 34 *<br>--- | 1-4,6, 12-15 | |
| | | | **TECHNICAL FIELDS SEARCHED (Int.Cl.7)** |
| E | EP 1 378 255 A (ETHICON INC) 7 January 2004 (2004-01-07)<br>The whole document<br>----- | 1-15 | A61L |

The present search report has been drawn up for all claims

| Place of search | Date of completion of the search | Examiner |
|---|---|---|
| THE HAGUE | 25 March 2004 | Menidjel, R |

CATEGORY OF CITED DOCUMENTS

X : particularly relevant if taken alone
Y : particularly relevant if combined with another *document of the same category*
A : technological background
O : non-written disclosure
P : intermediate document

T : theory or principle underlying the invention
E : earlier patent document, but published on, or after the filing date
D : document cited in the application
L : *document cited for other reasons*
& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 25 4080

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2004

| Patent document cited in search report | Publication date | Patent family member(s) | Publication date |
|---|---|---|---|
| US 3868955 A | 04-03-1975 | AT 338980 B | 26-09-1977 |
| | | BE 824345 A1 | 14-07-1975 |
| | | DE 2500162 A1 | 08-07-1976 |
| | | FR 2297636 A1 | 13-08-1976 |
| | | GB 1477571 A | 22-06-1977 |
| | | LU 71650 A1 | 24-06-1975 |
| | | NL 7500428 A ,B, | 16-07-1976 |
| US 3364200 A | 16-01-1968 | CH 430049 A | 15-02-1967 |
| | | FI 41060 B | 30-04-1969 |
| | | FR 1310496 A | 30-11-1962 |
| | | GB 983073 A | 10-02-1965 |
| | | NL 262878 A | |
| | | SE 301213 B | 27-05-1968 |
| US 4626253 A | 02-12-1986 | AT 66155 T | 15-08-1991 |
| | | AU 580337 B2 | 12-01-1989 |
| | | AU 4835685 A | 10-04-1986 |
| | | CA 1253321 A1 | 02-05-1989 |
| | | DE 3583786 D1 | 19-09-1991 |
| | | EP 0177064 A2 | 09-04-1986 |
| | | HK 17392 A | 13-03-1992 |
| | | IE 57311 B1 | 15-07-1992 |
| | | JP 1866569 C | 26-08-1994 |
| | | JP 5076310 B | 22-10-1993 |
| | | JP 61090668 A | 08-05-1986 |
| | | NZ 213571 A | 30-05-1988 |
| | | SG 107791 G | 16-04-1992 |
| | | ZA 8507682 A | 27-05-1987 |
| EP 1378255 A | 07-01-2004 | US 2004001879 A1 | 01-01-2004 |
| | | CA 2433968 A1 | 28-12-2003 |
| | | EP 1378255 A2 | 07-01-2004 |
| | | US 2004005350 A1 | 08-01-2004 |

EPO FORM P0459

For more details about this annex : see Official Journal of the European Patent Office, No. 12/82